# EUROPEAN PATENT APPLICATION

(11) **EP 1 938 753 A1**
(43) Date of publication of application: **02.07.2008**
(21) Application number: 07024919.8
(22) Date of filing: 21.12.2007
(51) Int. Cl.: A61B 8/00

(54) **Device for rotating a transducer of an ultrasonic probe**

(30) Priority: 27.12.2006 KR 20060134867
(71) Applicant: MEDISON CO., LTD., Kangwon-do 250-870 (KR)
(72) Inventor: Kim, Seong Rae, Gangnam-gu Seoul 135-280 (KR)
(74) Representative: Lorenz, Markus

(57) **Abstract**

Embodiments of the present invention may provide a device for rotating a transducer of an ultrasonic probe using a rod. The device of the present invention comprises: a drive motor; a drive disk driven by the drive motor; a transducer including a plurality of ultrasonic elements and having a rotating shaft; a rod holder mounted on the transducer and being shaped of a circular arc centering the rotating shaft of the transducer; and a rod jointed to the drive disk at its one end and to the rod holder at its other end. The rod is curved with a curvature larger than that of the rod holder.

## Description

The present application claims priority from Korean Patent Application No. 10-2006-0134867 filed on December 27, 2006, the entire subject matter of which is incorporated herein by reference.

### BACKGROUND

### 1. Field

The present invention generally relates to a device for rotating a transducer provided in an ultrasonic probe for use with an ultrasonic diagnostic apparatus.

### 2. Background

An ultrasonic diagnostic apparatus is widely used to diagnose a subject by visualizing a portion of the subject's body. For example, the ultrasonic diagnostic apparatus diagnoses a subject by detecting the alien substances of organs, measuring the level of a lesion, or observing a tumor or a fetus. Such ultrasonic diagnostic apparatus generally employ various ultrasonic probes in order to obtain information on a subject's body. The ultrasonic probe has a transducer, which emits ultrasonic waves into an inspection portion of a subject and receives the reflected ultrasonic waves therefrom to convert the reflected ultrasonic waves into electric signals. The ultrasonic diagnostic apparatus processes the electric signals from the ultrasonic probe, thereby forming ultrasonic images which show the inspection portion of a subject's body. In the recent years, an ultrasonic probe configured to rotate the transducer is used to obtain more accurate or three-dimensional ultrasonic images.

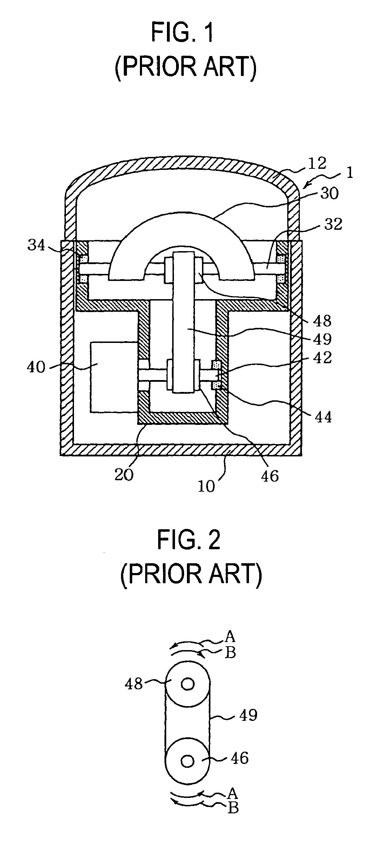

One example of a prior art ultrasonic probe for three-dimensional image is shown in Figs. 1 and 2. Fig. 1 is a schematic sectional view of a prior art ultrasonic probe for three-dimensional imaging. Fig. 2 is a schematic side view showing a power-transmitting arrangement of the ultrasonic probe shown in Fig. 1.

Referring to Fig. 1, the prior art ultrasonic probe 1 for three-dimensional imaging includes the following: a case 10; a base 20 mounted in the case 10; a transducer 30 comprised of a plurality of ultrasonic elements and having a rotating shaft 32; a drive motor 40 attached to the base 20 for driving the transducer 30; a transmission means for transmitting a drive force of the drive motor 40 to the transducer 30; and a cover 12 coupled to an upper end of the case 10 and being brought into contact with a skin of a subject to be inspected.

The rotating shaft 32 of the transducer 30 is horizontally disposed on the base 20 with its both ends rotatably supported by bearings 32 and 34. The drive motor 40 may be a servomotor (especially, a pulse motor). A drive shaft 42 of the drive motor 40 extends horizontally into the base and is supported by a bearing 44.

The transmission means includes: a drive pulley 46 coupled to the drive shaft 42; a driven pulley 48 coupled to the rotating shaft 32; and a belt 49 wound around the drive pulley 46 and the driven pulley 48. The belt 49 is a rectangular-sectioned flat strip.

Referring to Fig. 2, when the drive pulley 46 is rotated in a direction indicated by an arrow mark A, the driven pulley 48 is rotated in the direction A by the belt 49 as well. On the other hand, when the drive pulley 46 is rotated in a direction indicated by an arrow mark B, the driven pulley 48 is also rotated in the direction B by the belt 49. The rotating shaft 32 of the transducer 30 is coupled to the driven pulley 48. Thus, the transducer 30 can be reciprocated within a predetermined angular range along with the rotation of the driven pulley 48.

However, since slip can occur between each pulley 46 and 48 and the belt 49, the power output of the drive motor 40 cannot be fully converted into a force sufficient to rotate the transducer 30. Further, since vibrations of the drive motor 40 may be transferred to the transducer 30 via the belt 49 and the pulleys 46 and 48, the transducer 30 fails to work stably and consecutively, thereby causing diagnosis errors. Accordingly, there is a problem with the prior art ultrasonic probe in that the diagnosis errors occur much more frequently and the qualities of obtained ultrasonic images are deteriorated.

Further, if the belt 49 can be elongated due to repetitive operations, then the transducer 30 cannot be driven any further. In order to address such elongation of the belt 49, any belt tensioner may be provided in the ultrasonic probe 1, which in turn can unnecessarily enlarge a size of the ultrasonic probe 1. Further, since an expensive step motor is used as the drive motor 40, the manufacturing cost is relatively high. Accordingly, there is a further problem with the prior art ultrasonic probe 1 in that its durability is low and its manufacturing cost is high.

Consequently, there is a need to provide a device for rotating a transducer of an ultrasonic probe, which allows the transducer to be rotated smoothly and stably without any malfunction of the transducer.

### BRIEF DESCRIPTION OF THE DRAWINGS

Arrangements and embodiments may be described in detail with reference to the following drawings in which like reference numerals refer to like elements and wherein:

Fig. 1 is a sectional view of a prior art ultrasonic probe for three-dimensional imaging.

Fig. 2 is a schematic side view showing a power-transmitting arrangement of the ultrasonic probe shown in Fig. 1.

Figs. 3 to 6 are side views showing operations of a device for rotating a transducer of an ultrasonic probe, which is constructed in accordance with an embodiment of the present invention.

### DETAILED DESCRIPTION OF THE PRESENT INVENTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

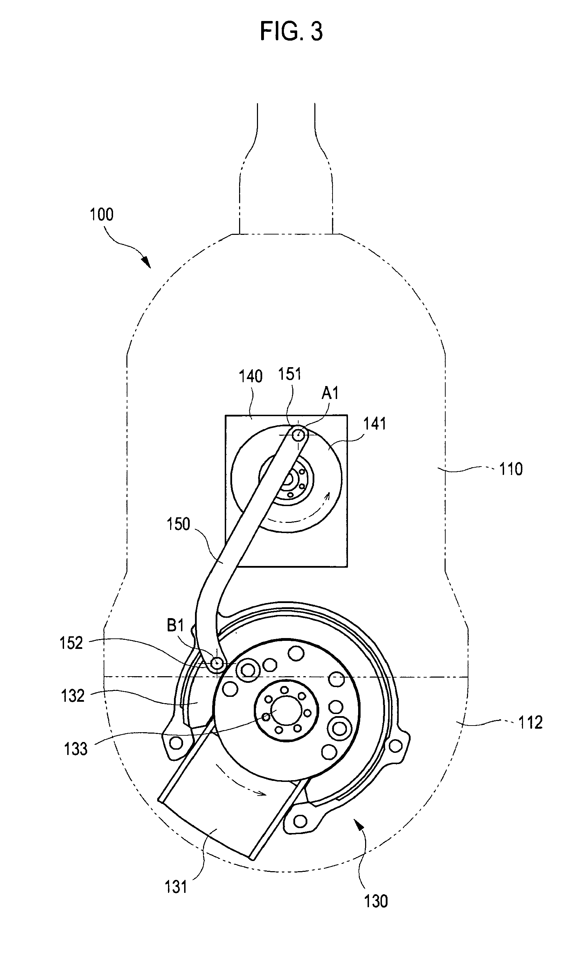

Figs. 3 to 6 are side views showing operations of a device for rotating a transducer of an ultrasonic probe, which is constructed in accordance with an embodiment of the present invention.

Referring to Figs. 3 to 6, a transducer 130 comprises a plurality of ultrasonic elements 131 arranged linearly or convexly. The transducer 130 is disposed inside a cover 112 of an ultrasonic probe 100. The transducer 130 includes a rotating shaft 133. The transducer 130 emits ultrasonic waves into an inspection portion of a subject from the ultrasonic elements 131 and receives the reflected ultrasonic waves while being rotated around the rotating shaft 133.

A drive motor 140 is disposed in a case 110 of the ultrasonic probe 100. A drive disk 141 is fixed to a drive shaft (not shown) of the drive motor 140.

A circular arc-shaped rod holder 132 is provided at the transducer 130. The rod holder 132 has a larger radius than that of the drive disk 141. The rod holder 132 is positioned so that its center coincides with a center of the rotating shaft 133 of the transducer 130. The rod holder 132 is disposed about the rotating shaft 133 with the ultrasonic elements 131 located between its both ends.

The drive disk 141 and the rod holder 132 are connected to each other via a rod 150. The rod 150 is shaped such that it is straight from its one end 151 to its middle portion and is curved with a certain curvature from its middle portion to its other end 152, as shown in Figs. 3 to 6. Alternatively, the rod 150 may be arcuately curved. The curvature of the curved portion of the rod 150 is equal to or larger than that of the circular arc-shaped rod holder 132.

The rod 150 is joined to the drive disk 141 (specifically, radially outward of the drive shaft of the drive motor 140) at the one end 151. The rod 150 is joined to the rod holder 132 (specifically, adjacent to one of both ends of the rod holder 132) at the other end 152. The rod 150 may be joined to the drive disk 141 and the rod holder 132 by a pin-joint, a rivet-joint, a bolt-and-nut-joint or the like.

In this embodiment, the drive motor 140 is a rotary motor. When the drive motor 140 rotates the drive disk 141, the one end 151 of the rod 150 is rotated in a rotation direction of the drive disk 141 along with the rotation of the drive disk 141. Then, the transducer 130 may be rotated around the rotating shaft 133 in the rotations direction of the drive disk 141 or in an opposite direction thereto by means of the rod 150 connecting the drive disk 141 and the transducer 130 (more specifically, the rod holder 132 provided at the transducer 130).

The drive motor 140 may be configured as a normally and reversely rotatable rotary motor. In such a case, the device for rotating a transducer of the present invention further includes: an encoder (not shown) coupled to the drive shaft of the drive motor 140 and a controller (not shown) for controlling a normal or reverse drive of the drive motor 140 to thereby control a rotational angle of the transducer 130. The encoder detects a rotational angle of the drive disk and converts it into digital signals. The controller controls the normal and reverse drive of the drive motor 140 based on the digital signals outputted from the encoder. With such constitution, a rotational range of the rotary motor (i.e., drive motor) can be measured by the encoder. Further, the rotary motor can be controlled to produce reverse rotation after accomplishing a desired or predetermined rotational range in one rotational direction.

A servomotor, a pulse motor or the like may be employed as the drive motor 140 instead of a rotary motor. In case the drive motor 140 is a pulse motor, the rotational angle of the transducer 130 may be controlled more accurately.

Referring back to Figs. 3 to 6, an operation of the device for rotating a transducer of the present invention will now be described.

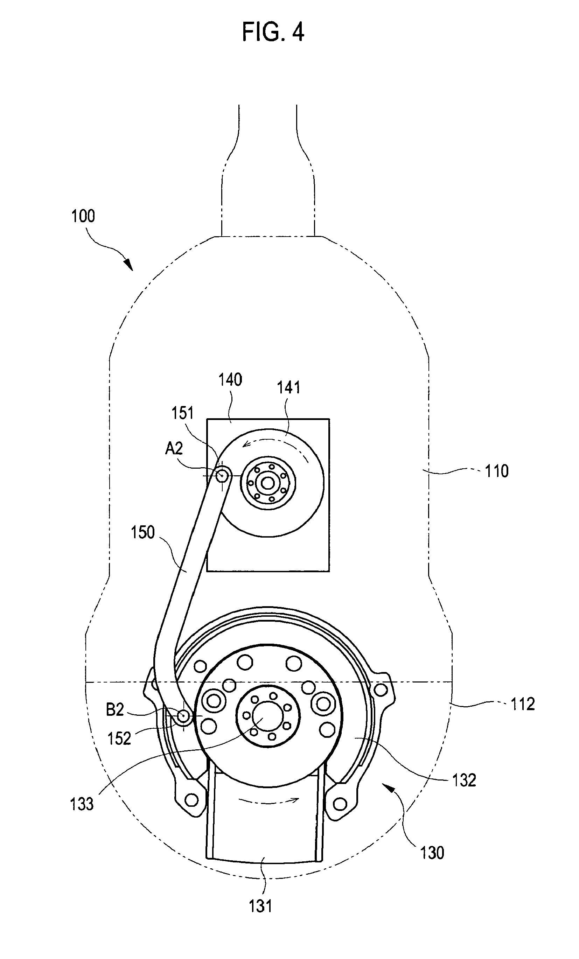

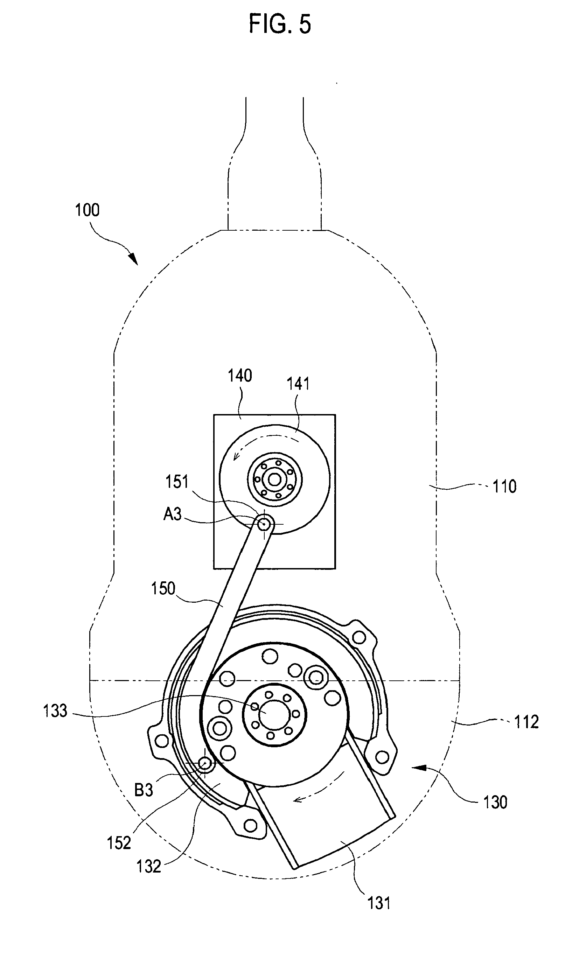

When the one end 151 of the rod 150 is circumferentially rotated along with the rotation of the drive disk 141 from a position A1 of Fig. 3 to a position A2 of Fig. 4, the other end 152 of the rod 150 is rotated accordingly around the rotating shaft 133 from a position B 1 of Fig. 3 to a position B2 of Fig. 4. Subsequently, when the one end 151 of the rod 150 is rotated from the position A2 of Fig. 4 to a position A3 of Fig. 5, the other end 152 of the rod 150 is rotated accordingly from the position B2 of Fig. 4 to a position B3 of Fig. 5.

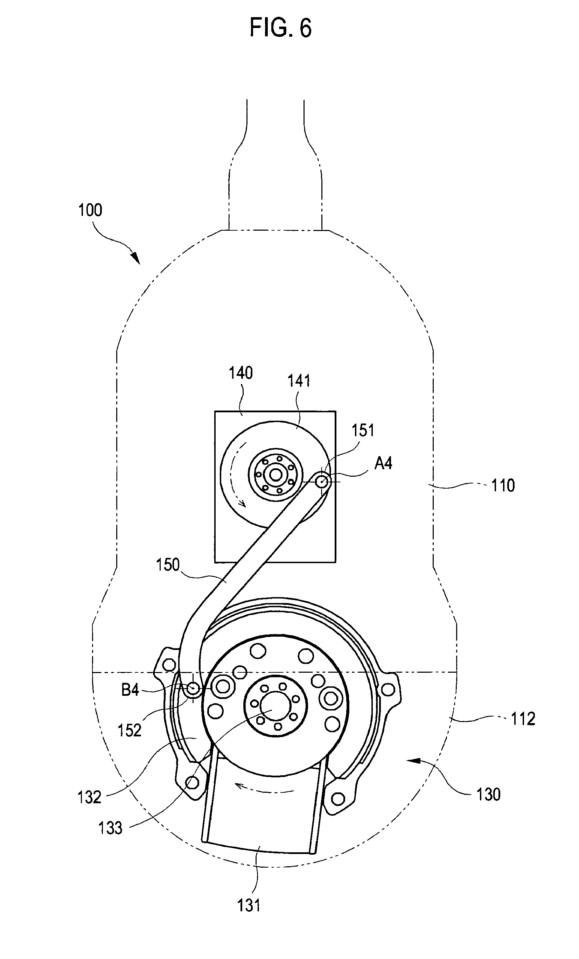

While the one end 151 of the rod 150 is rotated from the position A3 of Fig. 5 to a position A4 of Fig. 6, the other end 152 of the rod 150 is rotated from the position B3 of Fig. 5 to such a position that the transducer 130 is located at one limit of its rotational range and then the other end 152 of the rod 150 is reversely rotated from such a position to a position B4 of Fig. 6. Subsequently, when the one end 151 of the rod 150 is rotated from the position A4 of Fig. 6 to the position A1 of Fig. 3, the other end 152 of the rod 150 is rotated from the position B4 of Fig. 6 to the position B1 of Fig. 3.

In sum, as the one end 151 of the rod 150 is rotated going sequentially through the positions A1, A2, A3, A4 and A1, the other end 152 of the rod 150 is rotated going sequentially through the positions B1, B2, B3 B4 and B1 around the rotating shaft 133. This is because a length of the rod 150 is constant and the radius of curvature of the rod holder 132 is larger than that of the drive disk 141. During the above-described sequences, the transducer 130 is rotated around the rotating shaft 133 within a predetermined rotational range.

The rotational angle of the transducer 130 is the same as the rotational angle of the other end 152 of the rod 150. A rotational distance between the position B 1 and the position B2 is longer than that between the position B2 and the position B3. This means that the transducer 130 is decelerated during reversing its rotation. Thus, when the rotational direction of the transducer 130 is changed, impact to be applied to the transducer 130 can be reduced.

In case the radius of curvature of the rod holder 132 is smaller than the radius of the drive disk 141, the transducer 130 is not allowed to be rotated. This is because there can be a possibility that a distance between a portion of the drive disk 141 (to which the one end 151 of the rod 150 is coupled) and a portion of the rod holder 132 (to which the other end 152 of the rod 150 is coupled) is longer or shorter than the length of the rod 150. Further, even in case the radius of curvature of the rod holder 132 is equal to the radius of the drive disk 141, the transducer 130 is not allowed to be rotated. This is because the transducer 130 is rotated along with the rotation of the drive disk 141 without changing its rotational direction. Accordingly, it is preferable that the radius of curvature of the rod holder 132 is larger than the radius of the drive disk 141.

As discussed above, the device for rotating a transducer of the present invention uses a rod to convert a drive force of the drive motor into a rotation force of the transducer and employs a rotary motor as the drive motor. Thus, the transducer 130 can be rotated at a high speed with less vibration. Accordingly, a frame rate of the images acquired from the transducer 130 can be increased, and thus, high quality images can be obtained. Further, a stable rotation of the transducer 130 can be acquired.

Moreover, since impact applied to the transducer during change of the rotational direction does not nearly occur when compared to a prior art device with a step motor, the transducer can work more stably.

Further, since a rod is used as a device for rotating a transducer of the present invention instead of pulley, belts, etc., the ultrasonic probe can be configured more compactly and its manufacturing cost can be reduced.

Embodiments of the present invention may provide a device for rotating a transducer of an ultrasonic probe. The device may comprise: a drive motor having a drive shaft; a drive disk rotatable by the drive motor; a transducer including a plurality of ultrasonic elements, the transducer having a rotating shaft; a rod holder mounted on the transducer, the rod holder having a shape of a circular arc with its center at the rotating shaft of the transducer; and a rod rotatably jointed to the drive disk at one end thereof and to the rod holder at the other end thereof.

The drive motor may be a rotary motor.

The device may further comprise: an encoder coupled to the drive shaft of the drive motor for converting a rotational angle of the drive disk into a digital signal; and a controller for controlling a normal or reverse drive of the drive motor based on the digital signal from the encoder to control a rotational angle of the transducer.

The drive motor may be a pulse motor.

A radius of a rotational path of the one end of the rod may be shorter than a radius of a rotational path of the other end of the rod.

The rod may be curved with a curvature larger than that of the rod holder.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that various other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A device for rotating a transducer of an ultrasonic probe, comprising:
a drive motor having a drive shaft;
a drive disk rotatable by the drive motor;
a transducer including a plurality of ultrasonic elements, the transducer having a rotating shaft;
a rod holder mounted on the transducer, the rod holder having a shape of a circular arc with its center at the rotating shaft of the transducer; and
a rod rotatably jointed to the drive disk at one end thereof and to the rod holder at the other end thereof.

2. The device of Claim 1, wherein the drive motor includes a rotary motor.

3. The device of Claim 2, wherein the device further comprises:
an encoder coupled to the drive shaft of the drive motor for converting a rotational angle of the drive disk into a digital signal; and
a controller for controlling a normal or reverse drive of the drive motor based on the digital signal from the encoder to control a rotational angle of the transducer.

4. The device of Claim 1, wherein the drive motor includes a pulse motor.

5. The device of any one of Claims 1 to 4, wherein a radius of a rotational path of the one end of the rod is shorter than a radius of a rotational path of the other end of the rod.

6. The device of any one of Claims 1 to 4, wherein the rod is curved with a curvature larger than that of the rod holder.
